# EUROPEAN PATENT SPECIFICATION

(11) **EP 2 709 643 B1**
(45) Date of publication and mention of the grant of the patent: **16.05.2018**
(21) Application number: 12786044.3
(22) Date of filing: 18.05.2012
(51) Int. Cl.: A61K 36/889, A61P 27/02, A61K 31/05, A23L 33/105

(54) **COMPOSITIONS COMPRISING EXTRACTS OR MATERIALS DERIVED FROM PALM OIL VEGETATION LIGUOR FOR INHIBITION OF VISION LOSS DUE TO MACULAR DEGENERATION**
ZUSAMMENSETZUNGEN MIT EXTRAKTEN ODER MATERIALIEN AUS PALMÖLVEGETATIONSFLÜSSIGKEIT ZUR HEMMUNG VON SICHTVERLUST DURCH MAKULADEGENERATION
COMPOSITIONS COMPRENANT DES EXTRAITS OU DES MATIÈRES DÉRIVÉS DE LIQUEUR VÉGÉTALE D'HUILE DE PALME DESTINÉES À L'INHIBITION DE LA PERTE DE LA VUE DUE À LA DÉGÉNÉRATION MACULAIRE

(30) Priority: 18.05.2011 MY PI2011002220
(43) Date of publication of application: 26.03.2014
(73) Proprietor: Malaysian Palm Oil Board, Selangor Darul Ehsan (MY)
(72) Inventor: MOGHADAM, Ali Hafezi, Jaimaca Plain MA 02130 (US); SAMBANTHAMURTHI, Ravigadevi, 43000 Kajang Selangor Darul Ehsan (MY); TAN, Yew Ai, 43000 Kajang Selangor Darul Ehsan (MY); P MANICKAM, Kalyana Sundram, 43000 Kajang Selangor Darul Ehsan (MY); HAYES, Kenneth, C., Massachusetts (US); SOUSKA, Zandi, 10587 Berlin (DE)
(74) Representative: Pawlyn, Anthony Neil
(86) International application number: PCT/MY2012/000102
(87) International publication number: WO 2012/158018

(56) References cited:
- EP-A1- 1 230 923
- WO-A1-2010/126910
- WO-A1-2014/124140
- US-A1- 2007 243 211
- US-A1- 2009 226 547
- US-A1- 2009 252 817
- Carotech Bhd: "Tocomin, natural full spectrum palm tocotrienol/tocopherol complex.", Carotech Bhd, Malaysia , 10 May 2010 (2010-05-10), XP002729766, Retrieved from the Internet: URL:http://wayback.archive.org/web/2010051 0234107/http://www.carotech.net/index/prod uct_range/tocomin.html [retrieved on 2014-09-16]
- NAKAGAWA, KIYOTAKA ET AL.: 'In Vivo Angiogenesis Is Suppressed by Unsaturated Vitamin E, Tocotrienol' J. NUTR. vol. 137, no. 8, August 2007, pages 1938 - 1943, XP055131863

## Description

### FIELD OF INVENTION

The present invention relates generally to compositions based on oil palm plant and more particularly to compositions comprising materials obtained from palm oil vegetation liquor for the treatment and prevention of Macular degeneration.

### BACKGROUND

Reference to any prior art in this specification is not, and should not be taken as, an acknowledgement or any form of suggestion that this prior art forms part of the common general knowledge in Malaysia or any other countries.

Angiogenesis relates to the formation or sprouting of new blood vessels from preexisting vessels. Under normal circumstances, angiogenesis is observed as the formation of blood vessels during, but not limiting to, wound healing and embryonal development. For the past few years, great efforts have been expended by researchers to understanding the regulation of angiogenesis, identifying pathways to angiogenesis and much has been written about the mechanisms as well as its pathologic and physiologic conditions. There are three major processes or stages involved in the formation of blood vessels; whereby the first stage is angiogenic activation of endothelial cells and degradation of basement membrane, the second stage is endothelial proliferation and migration, and the third stage is new vessel formation.

Stages of maturation and stabilization of the newly formed blood vessels occur by means of recruitment of pericytes which may involve angiogenic factors such as vascular endothelial growth factor (VEGF). Accordingly, vascular endothelial growth factor (VEGF) plays a major role in all these three main stages. It facilitates in inducing angiogenic activated state of endothelial cells for the blood vessel, proliferation and migration of stimulus from the preexisting blood vessel and expression of integrins in new vessel formation.

One of the major causes of human blindness or adult vision loss is age-related maculopathy, especially in industrialized countries. Generally, such cause is associated to interference of normal physiological process of angiogenesis as described earlier, particularly due to local expansion of blood vessels, or uncontrolled angiogenesis.

An example of a leading cause of adult vision loss is age-related macular degeneration (AMD). Recent studies have demonstrated that AMD is caused by an assortment of clinically ocular findings that leads to vision impairment and blindness. Generally, AMD occurs in two forms, dry and wet. In wet AMD, choroidal vessels pathologically grow through the retinal pigmented epithelial (RPE) cell layer into the subretinal space, a process known as choroidal neovascularization (CNV) . According to scientific reports, CNV and ensuing leakage damage to the RPE and retinal cells would lead to permanent vision loss

Because VEGF is an important biomarker in angiongenesis (Zahir K Otrock, 2010) in many cases, VEGF has been identified as the key molecule responsible for the growth and leakiness of CNV, and the primary regulatory factor of neovascularization of angiogenic diseases. It can be concluded that VEGF, is one of the most significant factors affecting endothelial cell (EC) proliferation, among others, its motility and vascular permeability.

Macrophages are a major source of VEGF and tumor necrosis factor (TNF) -□, thereby it is implicated in the pathogenesis of AMD due to their spatiotemporal distribution in the proximity of the CNV lesions particularly in experimental models and humans.

In US 2007203211 A1, there is disclosed a drug for use in preventing or treating angiogenic eye diseases, in which the
method involves administering to a mammal in need thereof a pharmaceutically effective amount of angiotesin II receptor antagonist. It is further disclosed that angiotensin II receptor antagonist is highly effective in the prevention or treatment of intraocular angiogenic diseases such as proliferative retinopathy or retinal vein occlusion. There is no explicit disclosure on using plant based materials as VEGF inhibitors.

A great majority of treatments and medications are chemical based or surgical based treatments which may not be favorable for patients at advanced ages. Further, it has been shown that treatment for AMD is effective for only a small proportion of patients, particularly patients who have well-defined choroidal neovascular membrane (CNVM) (Bressler et al, 2004).

Therefore, there is a need to identify a solution and effective treatment for ocular angiogenesis and thus inhibits human blindness derived from highly abundant sources, such as plant based materials.

It is primary object of the present invention to provide a composition for use in the prevention and inhibition of vision loss due to Macular degeneration.

Still other objects of the present invention will become readily apparent to those skilled in the art from the following detailed description.

### SUMMARY OF INVENTION

The present invention relates to the prevention and inhibition of vision loss due to Macular degeneration by a composition comprising palm fruit juice.

In accordance with the present disclosure there is provided a composition comprising materials obtained from palm oil vegetation liquor, used for prevention and inhibition of vision loss due to ocular angiogenesis diseases.

In accordance with the present disclosure, the composition may be used in a method for inhibiting vision loss due to angiogenesis mediated or associated diseases, for instance but not limiting to ocular neovascularization, macular degeneration or any diseases where inhibition of angiogenesis is required.

In an additional aspect, the composition of the present disclosure may be used in a method for prevention of vision loss due to diseases associated with angiogenesis and VEGF receptor phosphorylation.

### BRIEF DESCRIPTION OF DRAWINGS

Some figures contain color representations or entities in order to elucidate the results of experiments for the purpose of the present invention.
FIG 1 (a) and (b) shows the effect of palm fruit juice (PFJ) of the present invention on CNV formation;
FIG 2 (a) and (b) shows the macrophage infiltration in CNV in accordance with a preferred method of the present invention;
FIG 3 (a) and (b) shows the leucocyte activation in experimental AMD;
FIG. 4 (a) and (b) shows tissues areas of angiogenesis in corneas;
FIG 5 shows the western blot of IKβ-α, IKB-α, pNFKB and NFKB in experimental AMD with and without palm fruit juice treatment.

### DETAILED DESCRIPTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Embodiments of the invention are described by way of illustration. As will be realized, the invention is capable of other and different embodiments and its several details are capable of modifications in various respects, all without departing from the scope of the present invention.

In accordance with the present disclosure, the composition comprises materials or extracts obtained from palm oil vegetation liquors, whereby said vegetation liquors maybe obtainable either directly from plants or as waste streams or aqueous streams in the processing of plant material. The vegetation liquors may be obtained based on wastes from any processing stages of oil palm at a palm oil mill.

Further in accordance with the present disclosure, the materials or extracts obtained from palm oil vegetation liquor of palm oil milling process are water soluble.

The materials or extracts obtained from palm oil vegetation liquor of the present disclosure contains phenolics.

The present disclosure provides a composition and method thereof for use in the treatment of ocular angiogenesis, and in particular age-related macular degeneration (AMD), whereby said composition is based on oil palm plants of the species *Elaeis.*

The present disclosure thereby introduces a novel composition and a novel method of medical treatment.

A particular aspect of the present disclosure contemplates a method for preparing a medicament based on a composition comprising materials obtained from vegetation liquors, for instance, palm fruit juice, in the treatment of an ocular angiogenesis.

Yet another aspect of the present invention provides a composition comprising extracts, more particularly palm fruit juice obtained from vegetation liquor for use in the treatment of an ocular angiogenesis, and more particularly, a macular degeneration associated diseases, such as, but not limiting to age-related macular degeneration (AMD).

Throughout the specification, the term *"Elaeis* sp" which may be used includes *Elaeis guineensis* and *Elaeis oleifera.*

As a preliminary example, it is described below the effects of extracts derived from oil palm vegetation liquor against CNV; more particularly said extract is palm fruit juice.

### Materials and Methods

In order to examine the effects of PFJ against vision loss, several experiments will be elucidated as examples were carried out and the results obtained will be described herein.

The palm fruit juice (PFJ) may be obtained by conventional means, from pure palm nut. The juice for use in the purpose of the present invention may contain other components however not to disrupt the nutritional content of fruit juice.

According to the present invention, the Palm Fruit Juice (PFJ) provides significant decrease in VEGF-A-induced angiogenesis. Such effect can be examined with a standard method of corneal micropocket assay as provided in EXAMPLE 1 below.

### EXAMPLE 1

### Corneal Micropocket Assay

Balb/C mice were anesthetized with an intraperitoneal injection of ketamine (100mg/kg) and xylazine (10mg/kg). Hydron pellets (0.3µl) containing 25, 100, 200, 400 or 1600ng human VEGF-A (293-VE; R&D Systems), 200 ng mouse VEGF-A (493-MV; R&D systems), were prepared and implanted into the corneas. Pellets were positioned in around 1.0±0.2mm distance to the corneal limbus. After implantation, bacitracin ophthalmic ointment (E. Fougera & Co) was applied to each eye to prevent an infection. On the indicated days after the implantation, digital images of the corneal vessels were obtained and recorded using OpenLab software version 2.2.5 (Improvision Inc) with standardized illumination and contrast.

### EXAMPLE 2

### Quantification of Angiogenesis in Whole- Mount

The mice were perfused with rhodamin-ConA and the eyes were taken out, radial cuts were then made in the peripheral cornea to allow flat mounting on a glass slide using a mounting medium (TA-030-FM, Mountant Permaflour; Lab Vision Corporation). The flat-mounted tissues were examined by fluorescence microscopy and recorded using OpenLab software version 2.2.5 (Improvision Inc) with standardized illumination and contrast. The results obtained were recorded.

### EXAMPLE 3

### Laser-induced CNV

To induce CNV, C57BL/6 mice were anesthetized and pupils were dilated with 5% phenylephrine and 0.8% tropicamide. Using a 532-nm laser (Oculight GLx, Iridex, Mountain View, CA), a slit-lamp delivery system, and a cover glass as a contact lens, four spots (100mW, 50 pm, 100ms) were placed in each eye. The lesions were located at 3, 6, 9 and 12 o'clock meridians centered on the optic nerve head and located ∼2 to 3 disk diameter from the optic nerve head. Development of a bubble under laser confirmed the rupture of the Bruch's membrane. Eyes showing haemorrhage were excluded from experiments. The results obtained for this step were recorded.

In accordance with another preferred embodiment of the present invention, PFJ decreases the CNV size, whereby an analysis to evaluate this effect is shown in EXAMPLE 4 below:

### EXAMPLE 4

### Evaluation of CNV

Seven days after laser injury, the size of the CNV lesions was measured in choroidal flat mounts. Briefly, mice were anesthetized and perfused through the left ventricle with PBS, followed by 5 ml of fluorescein-labeled dextran (5 mg/ml, fluorescein isothiocyanate-dextran; Sigma Aldrich) in 1% gelatin. Anterior segment and retina were removed from the eyecup. The remaining RPE-choroid-sclera complex was flat mounted after relaxing radial incisions using Mounting Medium (TA-030-FM, Mountant Permafluor; Lab Vision Corporation) and coverslips. Micrographs of the choroidal complex were taken using a Confocal Microscope (Leica, Wetzlar, Germay). The magnitude of the CNV lesions was determined by measuring the hyperfluorescent area using Openlab Software (Improvision, Boston, MA). The results obtained for this step were accordingly recorded.

According to another preferred embodiment of the present invention, the PFJ decreases macrophage infiltration, whereby a standard method can be used to examine such effect, as elucidated in EXAMPLE 5 below.

### EXAMPLE 5

### Immune Histochemistry

On day 3 after laser injury 10µm frozen sections of the posterior segment were prepared. The sections were incubated with a mouse anti-F4/80 mAb (10µg/ml), and subsequently with the secondary antibody. Photomicrographs of the CNV lesions were taken and the number of F4/80 positive macrophages was counted.

### EXAMPLE 6

### Western Blot

To obtain tissues, animals were perfused with PBS and eyes were enucleated immediately after perfusion. Choroid was microsurgically isolated and placed into 100µl of lysis buffer (mammalian cell lysis kit MCL 1, Sigma Chemical Co, St.Louis, MO), supplemented with protease and phosphatase inhibitors (Sigma), and sonicated. The lysate was centrifuged (12000 rpm, 15 min, 4°C) and the supernatant was collected. Each sample containing equal amount of total protein, quantified by protein assay (Bio-Rad Laboratories, Inc, CA) was separated by SDS-PAGE (sodium dodecyl sulfate-polyacrylamide gel electrophoresis), and electroblotted to PVDF (polyvinylidene fluoride) membranes (Invitrogen, Carlsbad, CA). To block the nonspecific binding the membranes were washed with 5% skim milk and subsequently incubated with a rabbit polyclonal antibody against (1µg/ml, Santa Cruz Biotechnology, Santa Cruz, CA) or a mAb against β-tubulin (1.5 µg/ml; Abcam, Cambridge, MA) at 4°C overnight, followed by incubation with a horseradish peroxidase-conjugated donkey or sheep antibody against rabbit or mouse IgG (1:2000; GE Healthcare UK limited Buckinghamshire, UK). The signals were visualized with chemiluminescence (ECL kit; GE Healthcare UK limited, Buckinghamshire,UK) according to the manufacturer's protocol.

### Statistics

All values are expressed as mean±SEM. Data were analyzed by t-test and differences between the experimental groups were considered statistically significant or highly significant, when the probability value, p, was <0.05 or <0.01, respectively.

### Results

Results obtained from each experiment were suitably recorded and analyzed via conventional means.

### I. Reduction of CNV size in experimental AMD using PFJ

To examine the potential of PFJ in reducing CNV, mice were fed PFJ for two weeks prior to laser injury. On day seven after CNV induction, CNV Volume was measured using confocal microscopy.

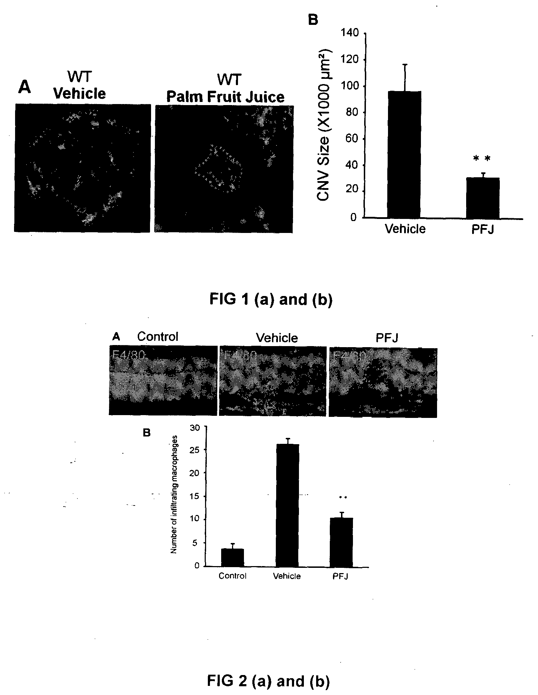

**FIG 1 (a) and (b)** shows the representative micrographs of CNV lesions in choroidal flat mounts from animals treated with vehicle or PFJ orally. Red dashed lines show the amount of CNV lesions filled with FITC-dextran. FIG 1(b) shows the quantitative analysis of CNV volume. As evident in FIG 1, it is observed that the CNV volume was reduced significantly compared to vehicle-fed controls.

### II. Reduction of microphage infiltration experimental AMD using PFJ

To investigate the effect of PFJ on macrophage infiltration in AMD, immune staining for the macrophage-specific marker, F4/80, and quantified the number of F4/80 positive cells in CNV lesion were performed. It is found and shown in **FIG 2 (a) and (b)** that macrophages were recruited to the CNV lesion one week after laser injury. In comparison, the number of accumulated macrophages at this time point was significantly reduced.

**FIG 2(a)** shows fluorescent micrograph of a laser induced CNV lesion, vehicle and treated with PFJ, immunostained with F4/80. The green shade shows the amount of macrophage infiltration with CNV lesion. **FIG 2 (b)** shows the quantitative analysis of macrophage infiltration. Bars show the average of macrophages number, n=3, P<0. 05.

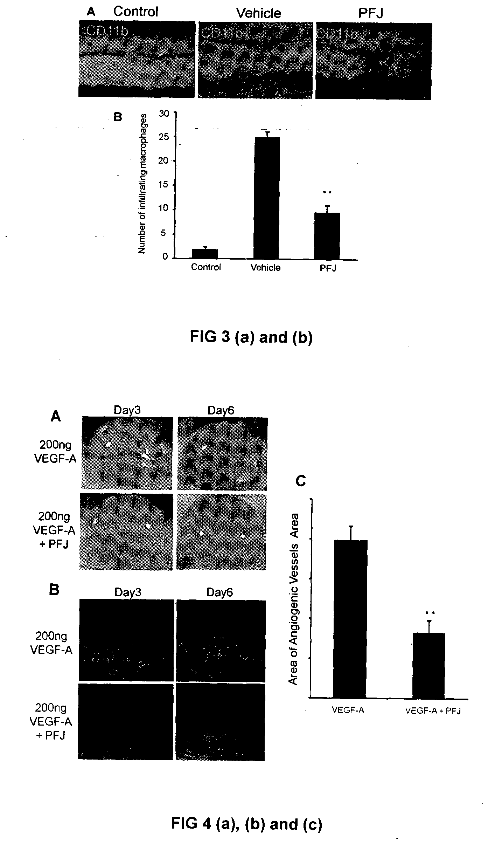

In order to investigate macrophage activation in the CNV lesion, immune staining was performed for CD11b, a leukocyte activation marker, and quantified the number of CD11b positive cells in CNV lesion. One week after laser injury it is observed that a significant number of activated immune cells accumulated in CNV lesions of vehicle fed animals. In contrast, it is observed that the number of activated leukocytes was significantly reduced in PFJ-fed animals. The results obtained based on this experiment were tabled and plotted as **FIG 3 (a) and (b)**.

**FIG 4(a)** shows the tissue areas of angiogenesis in corneas based on a preferred embodiment of the present invention. Digital images of the corneal vessels on 3^{rd} and 6^{th} day of VEGF-A implantation. Images in **FIG 4 (b)** shows the fluorescence microscopy of flat mounted cornea tissues, whereby on the 6^{th} day the mice were perfused with rhodamin-ConA.

**FIG 4 (c)** shows a plotted graph based on the quantitative analysis of the angiogenesis area. Based on this experiment, it is shown that the average of area (n=3), P<0.05.

### III. Suppression of pro-inflammatory signaling experimental AND using PFJ

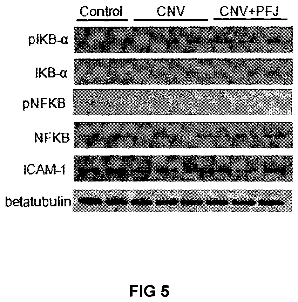

Investigation on the effect of PFJ on angiogenesis on a molecular level was performed based on implanted hVEGF-A (200ng) in corneas of mice that were fed vehicle or PFJ. To examine, whether PFJ reduces angiogenesis, on 3^{rd} and 6^{th} day after the implantation, digital images of the corneal vessels were obtained and subsequently quantified. Results based on this experiment are shown in **FIG. 5****,** wherein a western blot of plKB-□ , lKB-□, pNFKB and NFKB in. experimental AMD with and without Palm Fruit Juice (PFJ) treatment, three days after laser injury, while the control are unlasered eyes.

From the above, as it is widely known that immune cells and more particularly macrophages play a significant role in AMD pathology, disruption of monocyte recruitment and infiltration into ocular tissues may aid in preventing disease development and progression.

According to the present invention, it is shown in the experimental results that subjects fed or treated with Palm Fruit Juice (PFJ), exhibited:
a) Significant decrease in CNV size using confocal microscopy, in addition to reduction in macrophage infiltration using immune staining;
b) Significant decrease in VEGF-A-induced angiogenesis using established cornea pocket assay;
c) Inhibition of IKB-α phosphorylation in laser treated choroidal tissues with PFJ;
d) Reduction in macrophage recruitment to CNV lesions.

As briefly mentioned in earlier sections, in accordance with the present invention, the composition may be used in a method, for inhibiting vision loss due to angiogenesis mediated or associated diseases, for instance but not limiting to ocular neovascularization, macular degeneration or any diseases where inhibition of angiogenesis is required.

In another aspect of the present invention, the composition may be used for providing protective effect in diabetic retinopathy or damages to the eye's retina, various types of glaucoma which may lead to blindness or eyes disorders and corneal transplants.

Further, the composition of the present invention may be used in a method for prevention of vision loss due to diseases associated with angiogenesis and VEGF receptor phosphorylation.

Generally, the composition comprising palm fruit juice (PFJ) may be prepared in various suitable forms for direct or oral administration for the purpose of preventing adult vision loss.

According to the present invention, the PFJ of the present invention may be used to make supplements, or contained in drinks, edible products, tonics, health supplements, cosmetics. It is clear that PFJ may be prepared in concentrated form or extract.

Compositions suitable for oral administration may be presented in discrete units, such as capsules, sachets, lozenges, or tablets, each containing a pre-determined amount of the extract: as a powder or granules; as a solution or a suspension in an aqueous or non-aqueous liquid. Such compositions may be prepared by any suitable method of pharmacy which includes the step of bringing into association the extract of the present invention and one or more suitable carriers (which may contain one or more accessory ingredients as noted below). In general, the compositions of the invention are prepared by uniformly and intimately admixing the extract or any form of the palm fruit juice (PFJ) with a liquid or finely divided solid carrier, or both, and then, if necessary, shaping the resulting mixture. For example, a tablet may be prepared by compressing or moulding a powder or granules containing the extract or any form of the palm fruit juice (PFJ), optionally with one or more accessory ingredients.

Compressed tablets may be prepared by compressing in a suitable machine, the extracts in the form of a powder or granules, optionally mixed with a binder, lubricant, inert diluents, and/or surface active/dispersing agent (s). Moulded tablets may be made by moulding, in a suitable machine, the powdered compound moistened with an inert liquid binder.

Compositions may be prepared in a manner, and in a form/amount as is conveniently practiced.

As mentioned, the compositions of the invention may also be administered to a human in a dietary supplement form. Dietary supplements incorporating the active composition can be prepared by adding the composition to a food in the process of preparing the food. Any food may be used including, but not limited thereto, meats such as ground meats, emulsified meats and marinated meats; beverages such as nutritional beverages, sports beverages, protein fortified beverages, juices, milk, milk alternatives, and weight loss beverages; cheeses such as hard and soft cheeses, cream cheese, and cottage cheese; frozen desserts such as ice cream, ice milk, low fat frozen deserts, and non dairy frozen deserts; yoghurts; soaps; puddings; bakery products; salad dressings; and dips and spreads such as mayonnaise, butter, butter substitute, and other fat containing spreads. The composition is added to the food in an amount selected to deliver a desired dose of the composition to the consumer of the food.

Further, an effective amount of the compositions of the present invention is administered to a human subject. The actual dosage levels will depend upon a number of factors, such as specific mode of administration, the condition being treated, the condition of the patient and the judgement of the health care giver.

The composition comprising any form of the present invention may be prepared for use in a pharmaceutically effective or nutraceutically effective amount, solely on its own or in combination with other agents or compounds deemed appropriate by a person skilled in the art.

It is noted that the term 'pharmaceutically effective' and 'nutraceutically effective' amount includes a quantification that is acceptable for improving or prevention of vision loss, due to macular degeneration associated diseases.

Those skilled in the art will appreciate that the invention described herein is susceptible to variations and modifications other than those specifically described.

## Claims

1. A composition comprising an extract derived from palm oil vegetation liquor for use in preventing and inhibiting vision loss due to macular degeneration, wherein the extract is palm fruit juice.

2. A composition for use according to Claim 1, wherein the disease is age-related macular degeneration (AMD).

3. A composition for use according to Claim 1, which use involves inhibiting IKB-α phosphorylation in choroidal tissues.

4. A composition for use according to any of the preceding claims, wherein the extract contains phenolics.

5. A composition for use according to any of the preceding claims, wherein the composition is in the form of a dietary supplement.

6. A composition for use according to Claim 5, wherein the dietary supplement is a nutritional beverage.

7. A composition for use according to Claim 5, wherein the dietary supplement comprises a liquid or finely divided solid carrier, or both.

8. A composition according to Claim 5 or Claim 7, wherein the dietary supplement is prepared by adding the composition to a food or drink product, or adding the composition during the process of preparing a food or drink product.

## Patentansprüche

1. Zusammensetzung, umfassend einen Extrakt, der von Palmölpflanzenflüssigkeit abgeleitet ist, zur Verwendung bei der Verhinderung und Hemmung von Sehverlust aufgrund von Makuladegeneration, wobei der Extrakt Palmfruchtsaft ist.

2. Zusammensetzung zur Verwendung nach Anspruch 1, wobei die Erkrankung altersbedingte Makuladegeneration (AMD) ist.

3. Zusammensetzung zur Verwendung nach Anspruch 1, wobei die Verwendung das Hemmen der IKB-α-Phosphorylierung in Aderhautgeweben beinhaltet.

4. Zusammensetzung zur Verwendung nach einem der vorhergehenden Ansprüche, wobei der Extrakt Phenole enthält.

5. Zusammensetzung zur Verwendung nach einem der vorhergehenden Ansprüche, wobei die Zusammensetzung in Form eines Nahrungsergänzungsmittels vorliegt.

6. Zusammensetzung zur Verwendung nach Anspruch 5, wobei das Nahrungsergänzungsmittel ein Nährgetränk ist.

7. Zusammensetzung zur Verwendung nach Anspruch 5, wobei das Nahrungsergänzungsmittel eine Flüssigkeit oder einen fein verteilten festen Träger oder beides umfasst.

8. Zusammensetzung nach Anspruch 5 oder Anspruch 7, wobei das Nahrungsergänzungsmittel hergestellt wird, indem die Zusammensetzung einem Nahrungs- oder Getränkeprodukt zugegeben wird, oder indem die Zusammensetzung während des Herstellungsverfahrens eines Nahrungs- oder Getränkeprodukts zugegeben wird.

## Revendications

1. Composition comprenant un extrait dérivé d'eaux de végétation d'huile de palme, pour une utilisation dans la prévention et l'inhibition de la perte de vision due à la dégénérescence maculaire, où l'extrait est constitué de jus de fruit de palmier.

2. Composition pour une utilisation selon la revendication 1, où la maladie est la dégénérescence maculaire liée à l'âge (DMLA).

3. Composition pour une utilisation selon la revendication 1, laquelle utilisation implique l'inhibition de la phosphorylation d'IκBα dans les tissus choroïdiens.

4. Composition pour une utilisation selon l'une quelconque des revendications précédentes, où l'extrait contient des dérivés phénoliques.

5. Composition pour une utilisation selon l'une quelconque des revendications précédentes, où la composition se trouve sous la forme d'un complément alimentaire.

6. Composition pour une utilisation selon la revendication 5, où le complément alimentaire est une boisson nutritionnelle.

7. Composition pour une utilisation selon la revendication 5, où le complément alimentaire comprend un véhicule liquide ou solide finement divisé, ou les deux.

8. Composition selon la revendication 5 ou la revendication 7, où le complément alimentaire est préparé par l'addition de la composition à un produit d'aliment ou de boisson, ou l'addition de la composition lors du procédé de préparation d'un produit d'aliment ou de boisson.
